# EUROPEAN PATENT APPLICATION

(11) **EP 2 275 112 A1**
(43) Date of publication of application: **19.01.2011**
(21) Application number: 08873343.1
(22) Date of filing: 14.03.2008
(51) Int. Cl.: A61K 36/15, A61K 31/125, A61P 35/00

(54) **ANTITUMORAL TERPENOID PHARMACEUTICAL COMPOSITION 'ABISILIN' EXHIBITING ANGIOGENESIS-INHIBITING ACTION**

(71) Applicant: Obschestvo S Ogranichennoy Otvetstvennostyu "Initium-Pharm", Moskovskaya obl., 142000 (RU)
(72) Inventor: PINIGINA, Nina Maksimovna, Moscow 109469 (RU); LATSERUS, Ludmila Anatolievna, Moscow 109469 (RU); BARYSHNIKOV, Anatoly Urievich, Moscow 119607 (RU); MAGANOVA, Fania Irshatovna, Moscow 119991 (RU); KOZLOV, Aleksey Mihailovich, Moscow 115446 (RU); SMIRNOVNA, Zoya Sergeevna, Moscow 115580 (RU)
(74) Representative: Siegert, Georg
(86) International application number: PCT/RU2008/000147
(87) International publication number: WO 2009/113902

(57) **Abstract**

The invention relates to medicine and chemical and pharmaceutical industry, and concerns to a pharmaceutical for treating diseases associated with angiogenesis disorders, in particular, to a method for treating oncological diseases of various geneses, by means of inducing angiogenesis inhibition adjuvant to the direct antitumor, anti-recurrent, and antimetastatic effect, as well as the associated activation of endogenous apoptosis. The invention is accomplished by use of a new oral dosage form Abisilin containing, as ingredients, natural terpene compounds (isoprenoids) derived from coniferous trees of the Pinaceae family, this dosage form comprising: sesquiterpenoids (3 to 6%); neutral terpenoids (11 to 15%); diterpene acids (23 to 28%); triterpene acids (8 to 16%); unsaturated and saturated fatty acids (0.1 to 0.3%); phenolic compounds (0.1 to 0.2%); monoterpenoids being the rest, wherein the bornyl acetate content is at least 10.0% relative to the total terpenes composition. The use of the new oral dosage form Abisilin, the substances whereof are known to exhibit immunomodulatory, antibacterial, anti-inflammatory, pain-releasing, wound-healing, and other pharmacologically significant effects; which lacks counter-indications or toxic effects; and which is suitable for use together with various agents in a complex therapy, is intended to provide a new therapeutic approach and to enhance the efficiency of treating oncologic and many other diseases associated with angiogenesis disorders.

## Description

### Technical Field

The invention relates to medicine and chemical and pharmaceutical industry, in particular, to the development of an antitumor drug for treating oncologic diseases of various geneses via inducing a cascade of antitumor processes, comprising inhibition of angiogenesis, induction of endogenous apoptosis, slow down of tumor cell proliferation, and activation of antirecurrent and antimetastatic effects, by using a nontoxic multi-active agent inducing these processes, namely, the pharmaceutical drug composition Abisilin for oral administration based on terpenes (isoprenoids) from coniferous trees, the composition comprising the following components: monoterpenoids, sesquiterpenoids, neutral diterpenoids, diterpene acids, triterpene acids, phenolic compounds, and unsaturated and saturated fatty acids.

Further, the invention is useful for treating a plurality of diseases associated with angiogenesis disorders, primarily diseases caused by eye neovascularization (retinopathies or age-dependent macular degenerations), and for treating mesangial cell proliferative disorders in cases of chronic and acute kidney diseases (glomerulonephritis), psoriasis, progressive atheroma, arterial restenosis, diabetes, rheumatoid arthritis, chronic asthma, endometriosis, autoimmune diseases, arterial or post-transplantation atherosclerosis, and many other diseases.

### Background Art

The analysis of use of most antitumor drugs classified as chemotherapeutics, hormonal pharmaceuticals, or bioresponse modifiers, showed that most of them enhance tumor node regression while not affecting metastasis, but this is the most frequent cause of death in oncologic patients.

The possibility of inhibiting growth not only of a primary tumor but also of its metastases was discovered in a new type of antitumor pharmaceuticals, namely, anti-angiogenic pharmaceuticals, first proposed by Dr. Judas Folkman in 1971 (Folkman, J., Tumor Angiogenesis Therapeutic Implication, N. Engl. J. Med. 1971; 285: 1182-6). Anti-angiogenic pharmaceuticals have a number of advantages over conventional antitumor chemotherapeutics, namely: selective action, reduced risk of side effects, drug resistance, and reduced toxicity.

Anti-angiogenic pharmaceuticals by blocking tumor vascularization slow down or abort tumor growth and inhibit metastasis.

There is a plurality of ways for implementing this process, because the case in point is not a direct damage of cells with a specific regulatory mechanism; rather, this is the regulation of the activity of ordinary endothelial cells with a great many cell targets: inhibition of proliferation and/or migration, blocking of cell differentiation, neutralization of angiogenic growth factors (tyrosine kinase receptor CHER-1), action on vascular endothelial growth factor (VEGF) and the angiopoietin/TIF receptor system, inhibition of VEGFR-2 and VEGFR-3 angiogenic growth factors and their specific receptors, and inhibition of matrix metalloproteinases.

The normal functional reactivity of endothelial cells also makes itself vulnerable for the reason that its functioning is likewise determined by other cell systems of the body.

This plurality of targets allowed a number of potential anti-angiogenic inhibiting agents to put forward. For the prior-art anti-angiogenic pharmaceuticals, however, only some of the necessary advantages are noted.

For example, Avastin is known to be the most promising antitumor pharmaceutical; this is a drug of the monoclonal antibody pharmacologic group having an anti-angiogenic effect, capable of reducing vascularization and inhibiting tumor growth via selective binding with vascular endothelial growth factor (VEGF) (US patent no. 60542297; US patent no. 6639055).

However, the clinical efficacy of Avastin (which increases the total survival period from 15.6 to 20.3 months in patients bearing metastatic colorectal cancer against an increase from 13.6 to 17.7 months in patients treated by chemotherapy alone) stimulates search for more efficient pharmaceuticals. The more so as there is an impressive list of counter-indications and side effects for bevacizumab, the active substance of Avastin, which was used in a complex therapy of gastrointestinal perforation, hemorrhage, arterial thromboembolism, and other diseases.

Logically, angiogenesis inhibitors that block neovascularization-enhancing chemical signals were expected to be efficient against any type of tumor, as these are common processes.

The results of clinical tests of Avastin disprove these theoretical expectations.

There are also known combinations and compositions affecting the functions of the VEGP/VEGF receptor and angiopoietin/TIE receptor systems, capable of enhancing the inhibition of vascularization, and useful for treating cancer (RU 2292221, C2, June 20, 2001). These multiple compounds have an effect only on separate aspects of the multistep mechanism of the anti-angiogenic action, thereby only insignificantly affecting the enhancement of efficiency of an antitumor agent. Effects on the angiopoietin/Tie2 receptor system were studied to show only a slow-down of the growth of induced tumors (Siemeister et al., Cancer Res. 59, 3185-3191, 1999).

Anthranilic acid and thioanthranilic acid N-arylamides are known to inhibit the activity of VEGF tyrosine kinase receptor Fit-1 associated with neoplasmic diseases and with angiogenesis. Their use has an insignificant therapeutic efficiency.

The most pertinent art for the present invention as regards the biological activity spectrum and the source consists of Paclitaxel (Taxol), an antitumor pharmaceutical that efficiently inhibits endothelial cell proliferation, i.e., represents an angiogenesis inhibitor (Klauber, N., et al., Cancer Res., 57, 81-86, 1997).

Taxol was first developed on the basis of diterpenoids of complex structure, namely, taxanes which were isolated in 1971 from the bark material of Pacific Yew Tree (*Taxus brevifolia Nutl*)*.* Now, there are known methods for the complete synthesis of Taxol and its analogues (USA A1, No. 5488116, Jan. 2006; RU No. 2196581, May 4, 2000). Docetaxel (Taxotere), a semisynthetic analogue of Taxol, was isolated from the European Yew Tree (*Taxus baccata*) material, and R-tetraol was created using natural terpenoid complexes of galipot from coniferous trees of species *Pinus sibirica,* and/or species *Pinus cembra,* and/or genus *Abies,* and/or genus *Larix* (RU 2051900 C1, January 10, 1996), via isolation of the active ingredient from a natural product or via a semisynthetic route (RU 2196581, C2, May 4, 2000) using the labdanoid diterpene acids fraction admixed with camphor and exposing the resulting mixture to gamma-radiation to produce the R-tetraol structure.

The aforementioned compounds prepared by the above-specified methods have the following drawbacks: some degree of toxicity, which is intrinsic to all pharmaceuticals (causing neutropenia, fibrillar neutropenia, infections, vomiting, diarrhea, stomatitis, asthenia, neurologic (motion) and hematologic complications, etc.), and a low efficiency, these drawbacks impelling oncologists to search for combination schemes of such angiogenesis-inhibiting agents with other active agents for providing more efficient therapy.

These drawbacks of the prior-art anti-angiogenic terpenoid pharmaceuticals may stem from the multistep schemes proposed for their preparation, these schemes being targeted at isolating separate active substances in the pure form on the basis of partial or complete synthesis, which favors the appearance of various types of toxicities and deprives pharmaceuticals of the necessary multi-activity.

Thus, in spite of the existence of a plurality of targets for inhibiting angiogenesis and a number of prior-art pharmaceuticals, there is need in anti-angiogenic pharmaceutical compositions that would have all advantages intrinsic to the prior-art pharmaceuticals but would be free of the essential drawbacks thereof, these compositions being expected to:
- lack toxicity;
- lack counter-indications;
- reduce the toxicity and enhance the efficiency of antitumor cytotoxic agents when used in combination therewith (in need thereof);
- have direct antitumor activity;
- cause antirecurrent and antimetastatic effects;
- cause no resistance, specifically, to apoptosis;
- express neovascularization-inhibitory selectivity and abort neovascularization in tumor; and
- optionally, include immunomodulatory, pain-releasing, antibacterial, anti-inflammatory, and other types of activities in the multi-active spectrum of the anti-angiogenic agent;
- be capable of inducing cell apoptosis.

The above listing is far from exhausting the conditions necessary for reaching the required result in view of many drawbacks existing in prior-art methods and pharmaceuticals.

Anti-angiogenic agents capable of simultaneously causing such multi-active effects on tumor growth processes are neither known in nor obvious from the prior art.

### Summary of Invention

The purpose of the invention is to search for and develop pharmaceuticals for selective inhibition or complete aborting of neovascularization using an anti-angiogenic antitumor agent that is free of the extant drawbacks of the prior-art pharmaceuticals, namely, that does not cause any toxic side effects in long-term use, has no counter-indications, and does not cause resistance (specifically, to apoptosis) and that, when used in combination with cytostatic pharmaceuticals, not only enhances the efficiency thereof but also favors a reduction in the toxicity thereof. Further, the method claimed would have a number of advantages over all prior-art angiogenesis blocking agents, namely, it would have a direct antitumor effect, be capable of inducing cell apoptosis, and have antirecurrent and antimetastatic activities, simultaneously having immunomodulatory, antibacterial, wound-healing, anti-inflammatory, pain-releasing, and other activities.

Theoretically, complexes of natural terpenoids, being multicomponent and multi-active, might be expected to be free of the drawbacks intrinsic to the prior-art angiogenesis inhibition methods and to have some of the aforementioned necessary advantages.

The inventors of this invention were surprised, in the course of their scientific investigations, by the discovery that the terpenoid substance Abisil (described in patents RU No. 2054945, June 28, 1995; RU No. 2198653, March 29, 2002), which has immunomodulatory, anti-inflammatory, wound-healing, pain-releasing, and antibacterial activities and which is also capable of activating specific humoral mediators of nerve endings in the cerebral cortex in pathologic conditions (specifically, cancer) with neuromediator synthesis disorders (RU No. 2244928, February 19, 2003), is an efficient angiogenesis inhibitor, expressing cytotoxic activity, as well as apoptosis-inducing, antirecurrent, antimetastatic, and antitumor activities.

The Abisil terpenoid substance has served as the basis for a new pharmaceutical, namely, the multi-active terpenoid pharmaceutical composition Abisilin, which represents an oral dosage form, namely, a 20% oil solution, having a new spectrum of pharmaceutical activities and new opportunities for aborting pathologic processes associated with angiogenesis disorders, in particular, in malignant neoplasm growth.

The multi-activity of the terpenoid pharmaceutical composition Abisilin is accomplished due to the use of a natural chemical terpene complex containing a yield- and synthesis-provoked material that provides bioprotection against environmental impacts in representatives of the *Pinaceae* family, this material being enriched in identical monoterpene compounds. It was found that the enrichment of the natural terpene complex with separate most efficient agents (via direct extraction from the raw material or via induced native activation) did not give rise to toxic effects in use of the final product as intrinsic to the aforementioned prior-art anti-angiogenic pharmaceuticals. The high therapeutic effect is due not only to some superlative effect but also to the synergism of the effects of all active substances of the pharmaceutical composition Abisilin, namely: terpenes (isoprenoids) from coniferous trees of the family *Pinaceae,* comprising:
- sesquiterpenoids (3 to 6%);
- neutral terpenoids (11 to 15%);
- diterpene acids (23 to 28%);
- triterpene acids (8 to 16%);
- unsaturated and saturated fatty acids (0.1 to 0.3%);
- phenolic compounds (0.1 to 0.2%);
- monoterpenoids being the rest, wherein the bornyl acetate content is at least 10.0% relative to the total terpenes composition, dissolved in sunflower seed oil.

Further, the development of the antitumor terpenoid pharmaceutical composition Abisilin having an angiogenesis-inhibiting effect was based on a fundamentally new suggested strategy, which consisted in launching the production by brain structures of their own endogenous agents capable of blocking the misbalance in the regulation of normal physiological processes in a human or animal body, via using endogenous terpenoid compounds from coniferous plants synthesized therein to protect themselves against environmental impact.

The multi-active antitumor oral pharmaceutical Abisilin having an angiogenesis-inhibiting effect was studied for general toxicity.

In a preferred variation of the invention, proceeding from the general toxicity studies, Abisilin is administered orally in a dose of 80 to 100 mg per kilogram of body weight (see Example 1).

Another preferred variation of the invention is a method for inducing a selective cytotoxic effect on tumor cells and a method for killing them by an apoptosis-like mechanism using Abisilin in a dose of 1000 µg/mL, as for this dose the greatest proportion of cells positive for of AnV⁺PI⁻, AnV⁺PI⁻, and AnV⁻PI⁺ in total was found in respect of the Jurkat, cell line, which amounted to 86.6% (see Example 2).

One more preferred variation of the invention is a method for inducing the antitumor activity of Abisilin in the oral administration thereof in a dose of 10 to 100 mg per kilogram of body weight for a period of 2 to 11 days, as elucidated using a necessary kit of transplantable solid tumors: melanoma B-16, Ca-755, LLC, CC-5, CAC, and sarcoma M-1 (see Example 3).

The preferred variation of the invention representing treatment of tumor diseases of various geneses, consists of the use of the pharmaceutical composition Abisilin which may be in the form of a solution, tablets, gelatin capsules, pills, syrups, suspensions, powders, emulsions, granulates, microspheres or nanospheres, or other dosage forms that are useful for accomplishing a controlled release of the active ingredients of the pharmaceutical.

The administration of Abisilin together with the prior-art pharmaceutical Cisplatin which was studied on transplantable tumors CAC, Ca-755, and melanoma B-16, demonstrated not only the attainability of a synergistic effect from the use of Abisilin but also an enhancement of the therapeutic significance of both pharmaceuticals.

A preferred embodiment of the present invention is a method for displacing the natural balance between pro-angiogenic and anti-angiogenic mediators toward the dominance of the latter by using Abisilin *in vitro* and *in vivo* experiments (see Example 4).

Further, one more preferred embodiment of the present invention is the use of the pharmaceutical Abisilin for inducing antirecurrent and antimetastatic effects by the oral administration thereof in doses of 80 to 100 mg per kilogram of body weight (see Example 5).

Examples 1 to 5 are given below to illustrate the preferred embodiments of the present invention but do not limit the scope of the invention.

### Example 1

No death of animals was observed in determining acute toxicity in the maximal possible dose of 10 000 mg/kg, allowing the pharmaceutical to be classified into the Substance Hazard Category IV under the Russian State Standard (GOST) 12.1.007-76.

A subchronic experiment was carried out on non-pedigree male and female rats having a mean body weight of 165 ± 3 g and 163 ± 3 g, respectively. The studied pharmaceutical was administered intragastrically once a day for 14 days in a dose of 100, 500, or 1000 mg/kg to the male rats and in a dose of 100 and 1000 mg/kg to the female rats.

By the results of the experiment, Abisilin in the doses studied did not cause reliable changes in the integral indices of the test animals (body weight gain, behavioral reactions, food and water uptake); the cell composition of peripheral blood; the excretion, absorption, protein-synthesizing, and carbohydrate functions of the liver; the serum cholesterol level; and the functional condition of the cardiovascular, excretion, and nervous systems.

The use of Abisilin in a dose of 1000 mg/kg, i.e., a dose tenfold exceeding the recommended therapeutic dose, causes a weak local irritation of the gastrointestinal mucosa only in some of the test animals.

Thus, the general toxicity studies of Abisilin administration in the studied doses show that the use thereof does not cause structural disorders in body organs or tissues.

### Example 2.

### Cytotoxic and apoptosis-inducing activity of Abisilin toward tumor cells

Studies were carried out *in vitro* on the following tumor cell lines: Jurkat, (T-cell lymphoblastic leukemia), Raji (B-cell leukemia), K562 (chronic myelogenic leukemia), U937 (myeloleukemia), Mel P (melanoma), T 47 D (breast cancer), SKOV-3 (ovarian cancer), and PC-3 (prostatic cancer).

The cell lines were grown in the complete RPMI-1640 medium that contained 10% fetal calf serum, 2 mM/mL glutamine, 0.1 mg/mL gentamicin, vitamins, sodium pyruvate, and amino acids, at 37°C and in a CO₂ atmosphere.

In the studies Abisilin was used in the following concentrations: 300 µg/mL, 500 µg/mL, 800 µg/mL, and 1000 µg/mL. 1% DMSO (dimethyl sulfoxide) was used as a diluent.

The Abisilin cytotoxic activity was determined by a colorimetric method using the MTT assay. Tumor cells in a concentration of 5 × 10⁴ cells/mL were inoculated into Saarsted flat-bottomed 96-well plates, 20 µL Abisilin in a test concentration was added to each well, and the plates were incubated for 72 h under the standard temperature and pressure (each Abisilin concentration was studied in triplicate). The controls used were an Abisilin-free well triplet and a well triplet to which 20 µL of 1% DMSO was added. Five hours before the end of incubation, 20 µL of an MTT solution (from Sigma Chemical Co., U.S.A.) was added to each well. After the incubation was over, the cells were precipitated by centrifuging the plates at 1000 rpm for 2-3 min. The supernatant was carefully collected, and to each well added was 200 µL of DMSO (ICN Biomedicals, Inc.), which is a solvent for formazan crystals. The cells were resuspended and incubated for 10 min at 37°C, which was followed by measurements of the optical density (OD) of the solution on a Uniplan AIFR-01 immunoenzymatic reaction reader (Russia) at a wavelength of 540 nm. MTT is metabolized by living cells to water-insoluble formazan, thereby changing the optical density of the solution in proportion to the amount of surviving cells. Tumor cells were regarded as sensitive to the tested pharmaceutical if the amount of dead cells in the pool was at least 50%. Every experiment was performed in triplicate. The survival rate was determined as (mean OD in experiment/mean OD in control) × 100%.

Apoptotic cells were detected by double intravital staining using FITC-conjugated Annexin V in combination with the propidium iodide (PI) vital stain.

The Annexin V-FITC and PI stained cells were evaluated subject to the following criteria:
Annexin V negative/PI negative (AnV⁻ PI⁻) living cells, Annexin V positive/PI negative (AnV⁺PI⁻) early apoptotic cells, and Annexin V positive/PI positive (AnV⁺PI⁺) late apoptotic or necrotic cells. The control consisted of tumor cells incubated under the same conditions but in the absence of Abisilin, and tumor cells incubated under the same conditions but with 1% DMSO solution added instead of Abisilin.

The Statistika 6.0 software was used for statistical processing of the experimental data. The Student test was used to compare mean values (deviations were regarded as significant when p < 0.05).

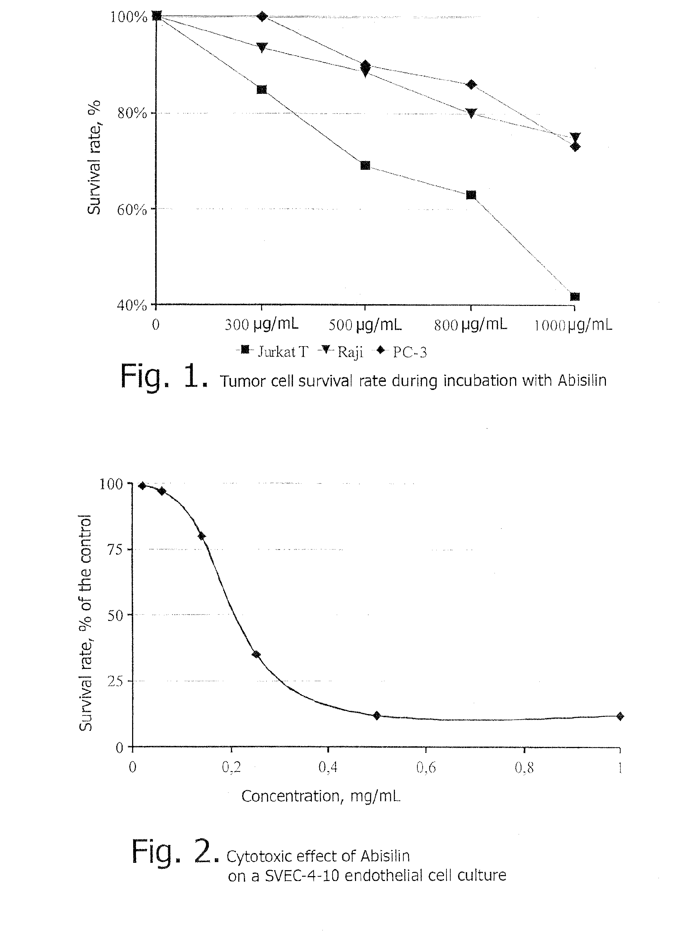

The studies show that the incubation of tumor cells with Abisilin reveals the cytotoxic activity thereof toward the Jurkat, Raji, and PC-3 cell lines. The tumor cell survival rate curves under Abisilin are shown in Fig. 1.

The most pronounced cytotoxicity of Abisilin was toward the Jurkat cell line and amounted to 41.8% for an Abisilin concentration of 1000 µg/mL. As the Abisilin concentration increased, the percentage content of living tumor cell decreased, and vice versa.

No cytotoxicity was detected in studying the Abisilin activity to U937, K562, T 47 D, SKOV-3, and Mel P tumor cells.

Abisilin-induced apoptosis was determined using Annexin V/PI double intravital staining on the Jurkat, Raji, PC-3, and U937 line cells for Abisilin concentrations of 300 µg and 1000 µg.

Abisilin was found to induce the death of tumor cells of the assayed lines by an apoptosis-like mechanism (Table 1).

**Table 1**

| Results of Abisilin-induced apoptosis studies | | | | |
|---|---|---|---|---|
| Agent | Overall proportion of tumor cells positive for AnV⁺PI⁻, AnV⁺PI⁺, AnV⁻PI⁺, % | | | |
| | Jurkat | Raji | PC - 3 | U 937 |
| Control (1% DMSO) | 26,0 | 23,1 | 33,1 | 1,7 |
| Abisilin, 300 µg/mL | 53,3* | 27,9 | 56,7 * | 7,4 |
| Abisilin, 1000 µg/mL | 86,6* | 44,9* | 62,4* | 38,2* |

| | | | | |
|---|---|---|---|---|
| Note: * p < 0.05, meaning a reliable deviation between the experiment and control. | | | | |

Noteworthy is the considerable increase in the number of apoptotic cells in response to increasing Abisilin concentration. The greatest proportion of cells positive for AnV⁺PI⁻, AnV⁺PI⁻, and AnV⁺PI⁻ in total was detected for the Jurkat cell line and was 86.6% for an Abisilin concentration of 1000 µg/mL.

Thus, the experimental data indicate the selective cytotoxic activity of Abisilin toward tumor cells and the ability of Abisilin to induce the death of these cells by an apoptosis-like mechanism thereby considerably enhancing the opportunities of antitumor therapy with use of anti-angiogenic agents.

Further, this aspect of the invention expands the application field of the known multi-active terpenoid substance Abisil and pharmaceutical compositions on the basis thereof recommended for use in topical administration and application, as the prior-art spectrum of effects (a combination of antibacterial, immunomodulatory, anti-inflammatory, wound-healing, and pain-releasing effects) is supplemented with some surprising and therapeutically significant effects, namely, the ability of causing cytotoxic effects on some tumor cell lines by an apoptosis-like mechanism.

This allows the prior-art composition Abisil and the composition Abisilin of the present invention to be recommended for use as agents capable of enhancing the efficiency of surgical treatment of tumor diseases.

### Example 3.

### Antitumor activity of the oral pharmaceutical composition Abisilin in transplantable tumors

The antitumor activity of Abisilin was studied on transplantable tumors in mice included in the list of obligatory animal tumor models used for selecting novel antitumor substances, namely: melanoma B-16, epidermoid Lewis lung carcinoma (LLC), breast adenocarcinoma Ca-755, stage 5 cervical cancer CC-5, colon adenocarcinoma (CAC), and polymorphic cell sarcoma M-1.

The experimental animals were BDF (C BI/6 × DBA/2) and F (C D1/6 and CBA/2) first-generation hybrid mice; CBA/2, BALB/c, and DBA/2 line mice (males and females), each animal weighing 20 to 25 g; and non-pedigree female rats, each animal weighing 200 to 250 g. The mice and rats were purchased from the Experimental Animal Division of the Blokhin Russian Oncologic Center, the Russian Academy of Medicinal Sciences, and maintained on an ordinary food ration.

Abisilin was administered orally (*per os*) in the form of an oil solution in doses of 10, 100, 150, 500, and 2500 mg/kg. Sunflower seed oil was used as the solvent for the pharmaceutical.

It was also studied whether the terpenoid pharmaceutical Abisilin is useful in combination with an antitumor agent from the group of wide-spectrum alkylating chemotherapeutic agents. Cisplatin purchased from Bristol-Myers Squibb was used in the experiments.

Abisilin was administered to animals orally every day for 10 days, or in the following schedule: on the 2nd and 6th days or on the 2nd and 11th days. The treatment was commenced 48 h after a solid tumor (Ca-755, CAC, melanoma B-16, LLC, CC-5, or M1) was transplanted. In the combined treatment, Abisilin was administered to mice orally every day for 10 days, beginning 48 h after tumor transplantation, in a dose of 50 mg/kg; the antitumor agent Cisplatin was administered once intraperitonially (ip) in a dose of 5 mg/kg. Animals bearing the aforementioned tumors were monitored until they died. The antitumor effects of the pharmaceutical were judged from percentage tumor growth inhibition (TGI, %) in the experimental animals against control animals.

The toxicity of the pharmaceutical was scored by the early death of mice against the death of control animals and by the condition of their organs (spleen, liver, and others) and the change in body weight relative to the initial value.

The statistical significance of the antitumor effect against the untreated control was determined by the Fisher-Student test. The difference between the compared groups was regarded as statistically reliable when p ≤ 0.05.

The results of studies of the Abisilin antitumor activity are shown below in Table 2.

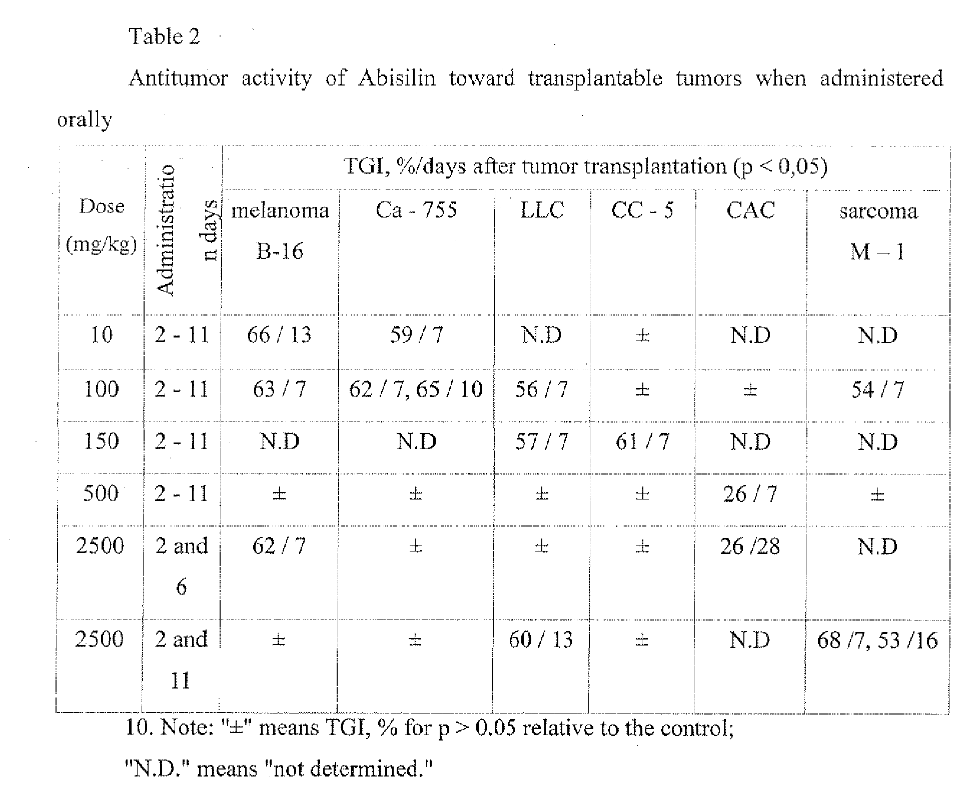

The studies showed that Abisilin had a short-term moderate antitumor effect on transplantable melanoma B-16 only in doses of 10 and 100 mg/kg when administered for 10 days: TGI = 66% (on day 13 of the experiment) and 63% (on day 7, p < 0.05), respectively, and also in a dose of 2500 mg/kg when administered twice with a 5-day interval (TGI = 62%) on day 7 of the experiment. However, the pharmaceutical was inefficient in a dose of 500 mg/kg when administered for 10 days or in a dose of 2500 mg/kg when administered twice with a 10-day interval.

In breast adenocarcinoma Ca-755, a statistically significant effect was detected in a dose of 10 mg/kg (TGI was 59% on day 7 of the experiment) and in a dose of 100 mg/kg (TGI = 62% on day 7 and 65% on day 10). In succeeding days, the antitumor activity systematically decreased.

On epidermoid Lewis lung carcinoma (LLC), the antitumor effect of the pharmaceutical of the present invention was detected in doses of 100 mg/kg and 150 mg/kg for oral administration on day 7 after tumor transplantation: TGI = 56% and 57%, respectively (p < 0.05). On the same tumor, Abisilin showed a moderate antitumor effect when administered in a dose of 2500 mg/kg twice with a 10-day interval: TGI = 60% on day 13 of the experiment.

The maximal tumor growth inhibition (TGI = 61%) for early cervical cancer (CC-5) in mice was observed on day 7 of administration in a dose of 150 mg/kg (TGI = 61%), that is, after the fivefold oral administration of the pharmaceutical.

In mouse colon adenocarcinoma (CAC), Abisilin was not efficient in doses of 50 mg/kg and 100 mg/kg in the 10-day administration schedule. However, the pharmaceutical showed a weak antitumor effect on day 7 of treatment in a dose of 500 mg/kg with 10-day administration (TGI = 26%) and on day 28 of treatment in a dose of 2500 mg/kg in the schedule of two infusions with a 5-day interval (TGI = 26%).

In transplantable rat sarcoma M-1, Abisilin showed a moderate antitumor effect directly after five administrations in a dose of 100 mg/kg on day 7 of the experiment: TGI = 54% (p < 0.05). When the pharmaceutical was administered twice (on days 2 and 11 after tumor transplantation) in a dose of 2500 mg/kg, an antitumor effect was also observed on days 7 and 16 from the commencement of treatment (TGI = 68% and 53%, respectively).

None of the experimental animals bearing transplantable tumors treated with Abisilin in the doses studied showed any manifestation of Abisilin toxicity. The therapeutic dose of Abisilin found in experiments on antitumor activity toward transplantable tumors was 100 mg/kg for every-day oral administration for 5 days.

The antitumor activity of Abisilin toward transplantable colon adenocarcinoma (CAC), breast adenocarcinoma Ca-775, and melanoma B-16 in mice was also studied in combination with the old antitumor pharmaceutical Cisplatin, both pharmaceuticals being administered in a dose equal to one-half the therapeutic dose used in monotherapy thereof (Table 3).

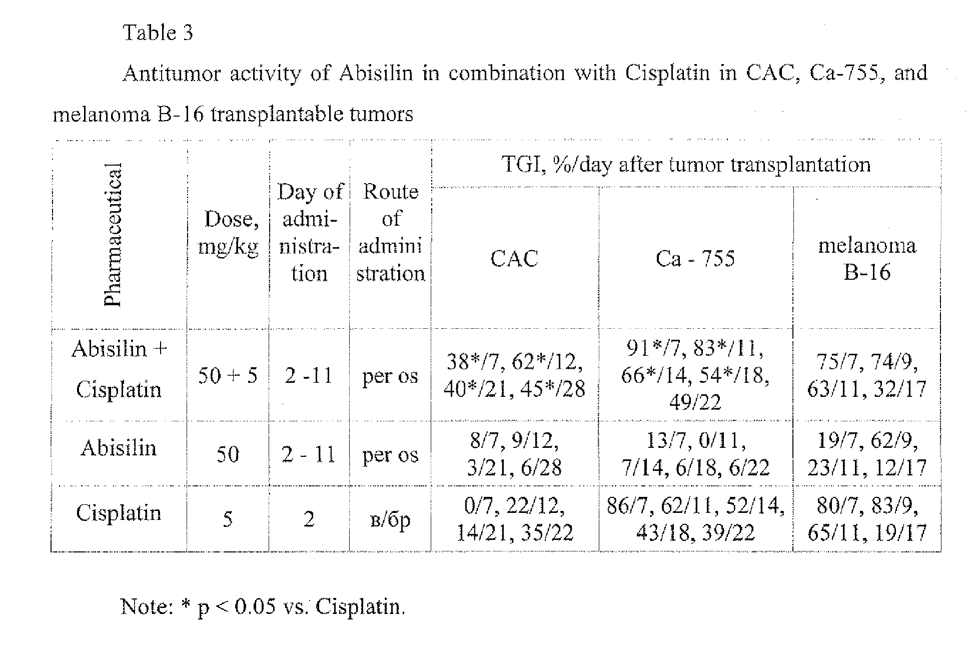

The data compiled in Table 3 make it clear that colon adenocarcinoma (CAC) is insensitive to Abisilin and Cisplatin in the monotherapy thereof. In the combined administration of these pharmaceuticals, a therapeutic effect was observed for 21 day (TGI = 38 to 62%).

For transplantable breast adenocarcinoma Ca-755, the combined use of Abisilin and Cisplatin resulted in a cumulative anticancer effect for 15 days: TGI = 91% against 86% after 7 days, 83% against 62% after 11 days, 66% against 52% after 14 days, 54% against 43% after 18 days, and 49% against 39% after 22 days. Further, the combined use of the two pharmaceuticals added up to 20% to the lifespan of experimental animals.

The combined administration of Abisilin and Cisplatin against transplantable melanoma B-16 in mice did not show an advantageous antitumor effect on this tumor relative to the Cisplatin monotherapy.

The moderate antitumor effect of Abisilin on five transplantable solid tumors of the six ones studied and the enhanced sensitivity of some tumors (CAC, Ca-755) to the combined administration of Abisilin with Cisplatin, gives grounds to suggest that Abisilin is useful for treating malignant tumors in combined chemotherapy in combination with other antitumor agents.

Thus, the experimental studies show that the multi-active terpenoid pharmaceutical Abisilin when administered orally has a moderate antitumor activity toward transplantable solid tumors (Ca-755, melanoma B-16, LLC, CC-5, and sarcoma M-1).

Further, it has been found that the combined administration of Abisilin and Cisplatin in one-half the therapeutic dose enhances the antitumor effect in experiments on animals bearing transplantable tumors.

The therapeutic Abisilin dose for every-day oral administration to animals bearing transplantable solid tumors was 100 mg/kg.

Abisilin did not cause toxic manifestations in a cumulative dose of 5 000 mg/kg regardless of whether it was administered every day in a dose of 500 mg/kg for 10 days or twice in a dose of 2500 mg/kg with intervals of 4 and 9 days.

### Example 4.

### In vitro and in vivo anti-angiogenic activity of Abisilin

The anti-angiogenic activity of Abisilin was studied on an SV40-virus-transformed mouse SVEC-4-10 endothelial cell pool [Walter-Yohrling, J., et al., Clin. Cancer Res. 2004; 10:2179-2189].

A stock solution of Abisilin having a concentration of 10 mg/mL (10% DMSO/PBS) was prepared in the Dosage Forms Laboratory of the Institute for Experimental Tumor Diagnostics and Therapy, Russian Oncologic Center, Russian Academy of Medical Sciences, within the span of the day of experiments. The stock solution was diluted with 1% PBS to the concentration required for the experiment.

Each experiment was repeated at least four times.

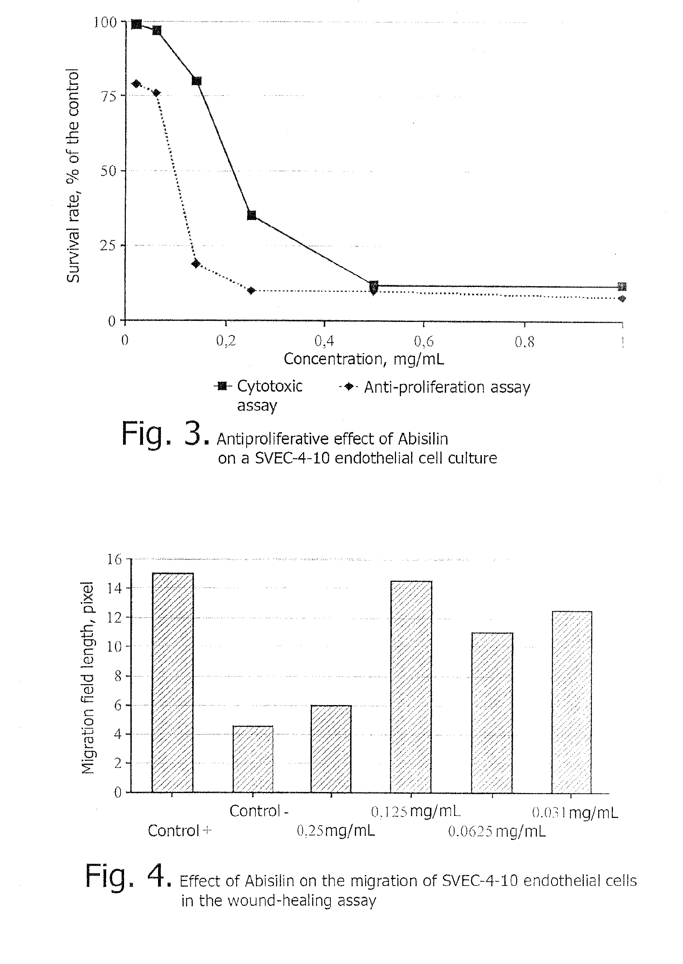

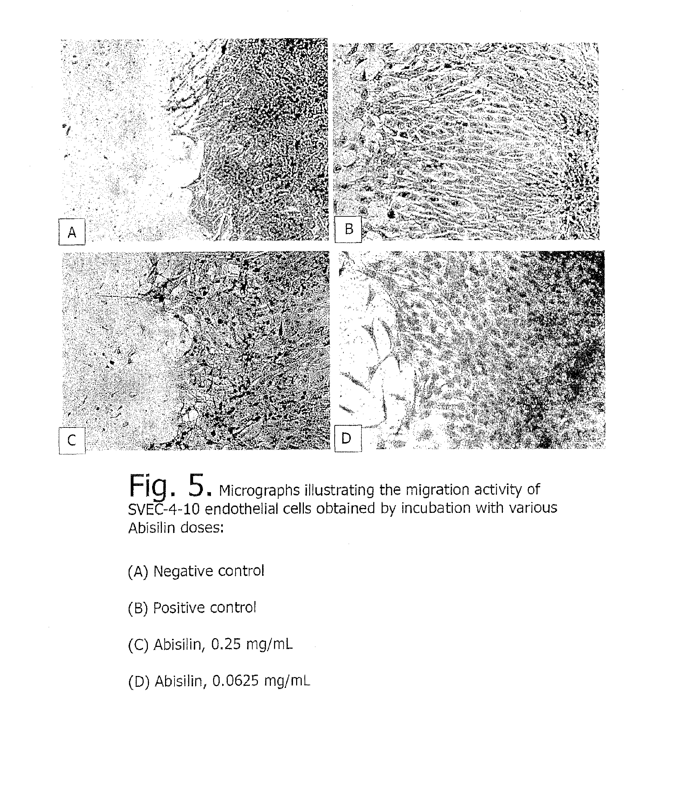

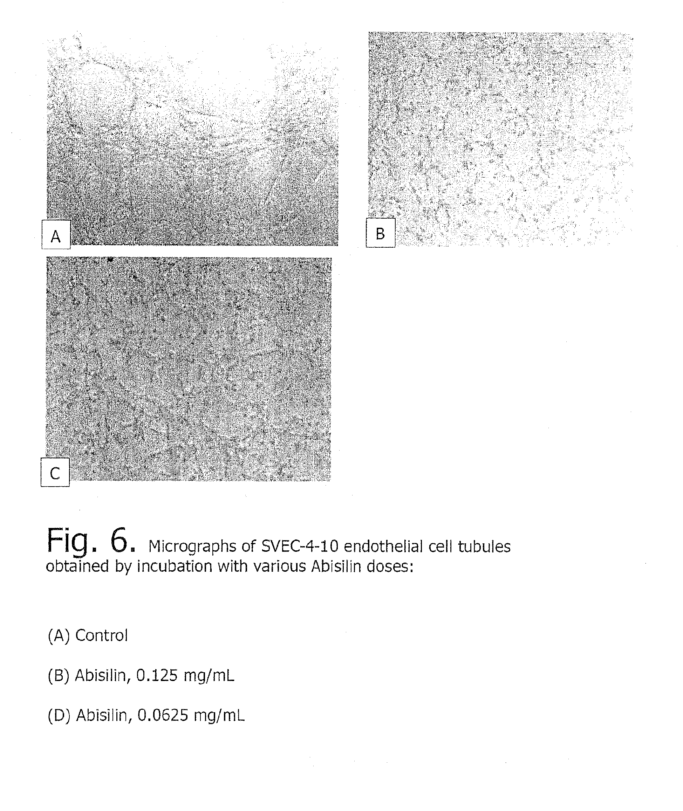

The anti-angiogenic activity of Abisilin may be demonstrated *in vitro* and *in vivo* using the following procedure:
(1) The cytotoxic effect of Abisilin on endothelial cells was caused by incubating the pharmaceutical for 24 h with SVEC-4-10 line cells inoculated at a high density.
   By our data, Abisilin kills endothelial cells over the range of concentrations from 1 mg/mL to 0.125 mg/mL (Fig. 2). FD₅₀ was 0.21 mg/mL.
(2) The antiproliferative effect of Abisilin was evaluated in studying the effect of Abisilin on the proliferative activity of bFGF-activated SVEC-4-10 endothelial cells inoculated at a low density.
   Abisilin was shown to inhibit the proliferation of SVEC-4-10 endothelial cells over the range of concentrations from 1 mg/mL to 0.125 mg/mL (IC₅₀ = 0.09 mg/mL).
   For two noncytotoxic concentrations (0.0625 mg/mL and 0.031 mg/mL), a 20% inhibition of endothelial cell proliferation is observed (Fig. 3).
(3) The antimigration effect of Abisilin was evaluated as the inhibition of migration of an SVEC-4-10 mouse endothelial cell pool (with use of wound area measurements) over the range of Abisilin concentrations from 0.25 mg/mL to 0.031 mg/mL (Figs. 4, 5).
   The 89% inhibition of in-wound migration of endothelial cells was observed for an Abisilin concentration of 0.25 mg/mL. At noncytotoxic concentrations (0.0625 mg/mL and 0.031 mg/mL), Abisilin does not inhibit in-wound migration of endothelial cells.
(4) Effect of Abisilin on tubular structures
   The ability of Abisilin to inhibit the formation of tubular structures by endothelial cells was ascertained for Abisilin doses in the range from 0.125 mg/mL to 0.015 mg/mL.
   Studies showed that Abisilin partially inhibited tubular formation in a concentration of 0.125 mg/mL. The tubules observed were short, open, and not confined by contacts with other tubules. For Abisilin concentrations of 0.0625, 0.031, and 0.15 mg/mL, no inhibition of tubular formation was observed (Fig. 6).
(5) Abisilin effect on angiogenesis in Matrigel implants
   The anti-angiogenic activity of Abisilin was studied *in vivo* as the angiogenesis-inhibitory ability thereof in Matrigel implants doped with a bFGF angiogenesis promoter. Abisilin was administered orally every day for 7 days in concentrations of 2, 1, and 0.2 mg/mL.

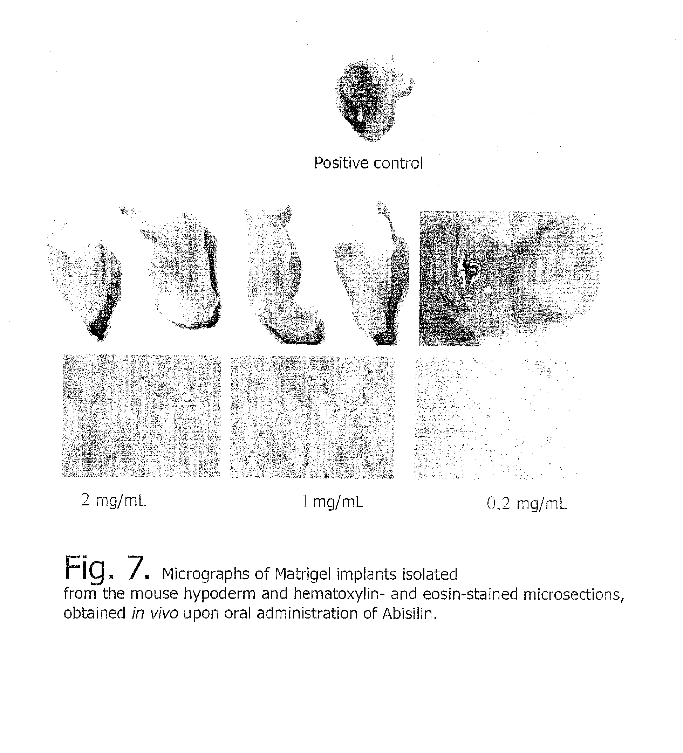

The studies showed that Abisilin in the studied concentrations dose-dependently inhibited vascularization in Matrigel (Fig. 7). The maximal inhibition was observed for Abisilin concentrations of 2 and 1 mg/mL; for 0.2 mg/mL, partial inhibition of neovascularization in the Matrigel implant was observed. Hematoxylin- and eosin-stained histological sections of the Matrigel implant showed a dose-dependent reduction of the amount of endothelial cells and stromal elements in mice fed with Abisilin.

Thus, Abisilin has been shown to have an anti-angiogenic activity *in vivo* and *in vitro.* The data obtained in these experiments are of applied significance which consists of the suitability of Abisilin for use in the claimed method as an agent capable of inhibiting angiogenesis in malignant tumors, the more so as methods for preparing such multi-active nontoxic agents simultaneously having an antitumor effect and an anti-angiogenic effect are yet unknown and unobvious in the art.

### Example 5.

### Antirecurrent and antimetastatic effects of Abisilin

The experimental development of a method for inhibiting metastasis and recurrence in malignant neoplasm employed C₅₇BL₆ line mice and BDF1 first-generation hybrids, each animal weighing 20 to 25 g, with subcutaneously implanted Lewis lung carcinoma. The mice were purchased from the Experimental Animals Laboratory Division of the Russian Oncologic Center, Russian Academy of Medicinal Sciences, and maintained on an ordinary food ration. All experimental procedures were carried out in accordance with international good practice for studies involving animals.

Abisilin was administered orally in the form of an oil solution in the dose range from 60 to 400 mg/mL. Sunflower seed oil was used as the solvent for the pharmaceutical. The control group and the treated group each comprised seven to ten animals.

The antitumor, antirecurrent, and antimetastatic activities of Abisilin were ascertained according to the recommendations recited in the Manual for Experimental (Preclinical) Study of Novel Pharmacological Substances published in 2005.

Student tests were used for data processing.

The effect of Abisilin on the rate and time of appearance of induced tumors and the dynamics of their progression were studied after recurrences were induced by means of transplantation of 1 × 10⁵ Lewis lung carcinoma cells underneath the stitch preliminarily put on a cut through skin in mouse,

The oral administration of Abisilin in doses of 100 mg/mL and 400 mg/mL for 10 days caused a well defined antirecurrent effect. For example, in a group of animals that was given Abisilin in a dose of 400 mg/mL after tumor cells were implanted, recurrences appeared in two of the seven animals only after 14 days, whereas in a group of animals that were given Abisilin in a dose of 100 mg/mL, recurrences appeared only in three of the seven animals by day 12 of the experiment. Moreover, in the other animals induced recurrences were not found during an observation period longer than 90 days.

The study of the effect of oral Abisilin administration (for 10 days in doses of 60, 120, and 360 mg/mL) on the growth and metastasis of induced recurrences, showed that not only did Abisilin administration inhibit the dynamics of appearance and growth of induced Lewis lung carcinoma but it also reduced metastasis of recurrent tumors by 46% when administered in a dose of 120 mg/mL (Table 4). Despite metastases were revealed in all treated and control animals after they were killed on the 33rd day, the mean mass of the lungs bearing metastatic cancer was 241.0 ± 36.0 mg compared to 449.7 ± 146.1 in the control animals.

The effect of Abisilin (administered in a dose of 60, 100, or 120 mg/mL; orally; fivefold; before, after, or before and after resection of the primary tumor node) on the spontaneous recurrence and metastasizing of Lewis lung carcinoma depended on the administration schedule thereof (Table 5).

In the event the pharmaceutical was administered in a dose of 60 mg/mL, the best effect was reached by adjuvant therapy after the resection. By day 27 of the experiment, recurrences appeared in 3/7 of the mice of the test group against 6/7 of the control group. The pre-surgery therapy and a combination (pre- and post-surgery) schedule were both less efficient. However, Abisilin administration in a dose of 120 mg/mL was most efficient in the pre-surgery administration schedule. After animals were killed on day 35 of an experiment intended to evaluate the effect of the therapy on the metastasis of Lewis lung carcinoma, data was obtained that post-surgery (adjuvant) therapy is also most efficient when Abisilin is used in a dose of 60 mg/mL. In animals that were given Abisilin according to this schedule (orally, 60 mg/mL dose, 5 days after resection of a primary tumor), 45% metastasis inhibition was noticed (the mean mass of metastatic-carcinoma-bearing lungs in treated animals was 393.3 ± 160.0 mg compared to 709.4 ± 237.3 mg in control animals).

**Table 4.**

| Effect of Abisilin on the dynamics of appearance, growth, and metastasis of induced recurrences of Lewis lung carcinoma in C57B1/6 mice | | | | | | | | | |
|---|---|---|---|---|---|---|---|---|---|
| N | Action | Drays after implantation (number of mice with recurrences) | | | Animals are killed on the 33rd day | | | | Metastasis rate |
| | | | | | recurrence | | lungs | | |
| | | | | | Mean weight | TGI | Mean weight | TMI | |
| | | 6 | 10 | 13 | g | % | mg | % | |
| 1 | Surgery + oil per os 0.1 mL/mouse × 10 (days 1-10) | 2/7 (29%) | 7/7 (100%) | 7/7 (100%) | 5,7±2,0 | | 449,7±146,1 | | 7/7 1+;5++;1+++ |
| 2 | Surgery + Abisilin per os 60 m/kg × 10(days 1-10) | 2/7 (29%) | 6/7 (86%) | 7/7 (100%) | 4,8±2,3 | 25 | 438,9±228,2 | 2 | 27/7 4+;1++;2+++ |
| 3 | Surgery + Abisilin per os 120 mL/kg × 10 (days 1-10) | 1/7 (14%) | 6/7 (86%) | 7/7 (100%) | 5,1±1,2 | 20 | 241,0±36,0 | 46 | 7/7 46 5+;2++ |
| 4 | Surgery + Abisilin per os 360 mL/kg + topically × 10 (days 1-10); | 4/7 (57%) | 7/7 (100%) | 7/7 (100%) | 4,5±2,8 | 30 | 406,0±41,0 | 10 | 7/7 1+;3++;3+++ |

| | | | | | | | | | |
|---|---|---|---|---|---|---|---|---|---|
| 16. Note: *The ratio of the number of animals with recurrences to the total number of animals in the group; (+) less than 10, (++) from 10 to 30, and (+++) more than 30 metastases. | | | | | | | | | |

**Table 5. Effect of Abisilin on the spontaneous recurrence and metastasis of Lewis lung carcinoma adjuvant to the surgical resection of primary tumors**

| Nº | Action | Days of experiment. Number of mice with recurrences | | | Animals are killed on day 35 | | | | Metastasis rate |
|---|---|---|---|---|---|---|---|---|---|
| | | | | | recurrence | | lungs | | |
| | | | | | Mean weight | TGI | Mean weight | TMI | |
| | | 14 | 19 | 27 | g | % | mg | % | |
| 1 | Oil per os 0.1mL/mouse × 5(days 3-7) + surgery (day7)+Oil per os 0.1mL/mouse×5(days 8-12) | 0/7 | 4/7 | 6/7 | 4,8±2,4 | | 709,4±237,3 | | 7/7 [1++;6+++] |
| 2 | Abisilin per os 60 mg/kg × 5 (days 3-7) + surgery (day 8) | 1/7 | 5/7 | | 5/7 5,7±2,1 | +18 | 883,6±352,7 | +25 | 7/7 [1++;6+++] |
| 3 | Abisilin per os 60 mg/kg × 5 (days 3-7) + surgery (day 7) + Abisilin per os 60 mg/kg × 5 (days 8-12) | 0/7 | 6/7 | 6/7 | 6,0 ± 1,8 | +25 | 471,7±167,8 | 34 | 6/6 [1++;5+++] |
| 4 | Surgery (day 7) + Abisilin per os 60 mg/kg × 5 (days 8-12) Surgery (day | 2/7 | 4/7 | 5/7 | 5,8±2,6 | +20 | 393,3±160,0 | 5 | 7/7 [4++;3+++] |
| 5 | 7) + Abisilin per os 60 mg/kg × 10 (days 8-17) | 0/7 | 3/7 | 3/7 | 5,3±3,5 | 11 | 560,4±338,3 | 21 | 7/7 [1+;1++;5+++] |
| 6 | Abisilin per os 120 mg/kg × 5 (days 3-7) + surgery (day 8) | 1/7 | 2/7 | 4/7 | 3,0±2,0 | 38 | 602,7±254,7 | 15 | 6/7 [1-;2++;4+++] |
| 7 | Abisilin per os 120 mg/kg × 5 (days 3-7) + surgery (day 7) + Abisilin per os 120 mg/kg × 5 (days 8-12) | 0/7 | 6/7 | 7/7 | 4,4±2,3 | 8 | 758,4±435,7 | +7 | [1+;2++; 4+++] |
| 8 | Surgery (day 7) + Abisilin per os 120 mg/kg × 5 (days 8-12) | 2/7 | 4/7 | 6/7 | 5,2±2,7 | +8 | 596,4±343,6 | 16 | 7/7 [2++;5+++] |
| 9 | Surgery (day7)+Abisilin per os 120 mg/kg × 10 (days 8-17) | 0/7 | 4/7 | 4/7 | 5,6±4,2 | +4 | 585,8±101,0 | 17 | 7/7 [3++,4+++] |

| | | | | | | | | | |
|---|---|---|---|---|---|---|---|---|---|
| Note: *The ratio of the number of animals with recurrences to the total number of animals in the group; (+) less than 10, (++) from 10 to 30, and (+++) more than 30 metastases. | | | | | | | | | |

It follows from the experimental data that the claimed method of use of the multi-active terpenoid pharmaceutical Abisilin in the set schedule and the oral dosage form, reaches the required result, namely: inhibits induced recurrence of Lexis lung carcinoma and suppresses (by 25 to 46% in various experiments) the metastasis of recurrent tumors. The best effect was reached by the oral administration of the pharmaceutical for the next 7 to 10 days in a dose of 100 to 120 mg/kg. Abisilin activity studies in adjuvant therapy after the surgical removal of a primary tumor revealed its ability to inhibit the appearance and progression of spontaneous recurrences of Lewis lung carcinoma. Most efficient was the use of the pharmaceutical in a dose of 60 mg/kg for 5 days after the surgical removal of a primary tumor. In a dose of 120 mg/kg, the pharmaceutical was more efficient in pre-surgery administration. The five-time post-surgery administration of Abisilin provided the 45% inhibition of Lewis lung carcinoma metastasis adjuvant to the surgical removal of the primary tumor.

Thus, the claimed method of oral administration of the multi-active terpenoid pharmaceutical Abisilin in the set schemes and schedules gave a surprising opportunity to inhibit the angiogenesis process in the malignant neoplasm progression, and to:
- cause antirecurrent and antimetastatic effects;
- have no counter-indications;
- cause cytotoxic and apoptosis-inducing effects;
- have antitumor activity in required strains of transplantable tumors in mouse;
- reduce the toxicity and enhance the therapeutic efficiency of cytotoxic antitumor agents (Cisplatin) when used in combination therewith;
- cause no resistance;
- be selective in inhibiting neovascularization;
- have no toxic effect in oral administration in doses tens of times surpassing the therapeutic dose.

Further, the pharmaceutical expands the field of use of the old multi-active terpenoid substance Abisil and the pharmaceutical compositions on the basis thereof recommended for topical administration and application as agents capable of enhancing the efficiency of the surgical treatment of tumor diseases.

Another cause of the high therapeutic effect of the pharmaceutical Abisilin is the previously known multi-activity of its Abisil substance, namely, a combination of immunomodulatory, antibacterial, wound-healing, anti-inflammatory, and pain-releasing activities, this substance further being capable of stimulating the synthesis of specific endogenous humoral mediators of nerve endings in the cerebral cortex.

The proposed method for the containment of malignant neoplasm gave an opportunity to accomplish an intended purpose, this purpose consisting in revealing and developing an agent for selective inhibition or aborting of neovascularization using the anti-angiogenic antitumor pharmaceutical Abisilin which is free of the drawbacks of the prior-art pharmaceuticals, namely, does not cause toxic side effects in long-term use, has no counter-indications, and does not cause resistance (specifically, to apoptosis), and which not only enhances the efficiency of cytostatic agents when used in complex therapy therewith but also favors the reduction of the toxicity thereof. Further, the pharmaceutical has some advantages over all prior-art angiogenesis-inhibiting agents, namely, it has the direct antitumor effect, can induce cell apoptosis, causes anti-recurrent and antimetastatic effect, at the same time having immunomodulatory, antibacterial, wound-healing, anti-inflammatory, pain-releasing, and other effects.

## Claims

1. A pharmaceutical for treating and preventing tumor and other diseases in which angiogenesis inhibition is a therapeutically effective action by using the multi-active pharmaceutical composition Abisilin, wherein the active ingredients are terpenes (isoprenoids) from coniferous trees of the *Pinaceae* family, these terpenes comprising the following components on percentage base relative to the total weight, namely:
- sesquiterpenoids (3 to 6%);
- neutral diterpenoids (11 to 15%);
- diterpene acids (23 to 28%);
- triterpene acids (8 to 16%);
- unsaturated and saturated fatty acids (0.1 to 0.3%);
- phenolic compounds (0.1 to 0.2%);
- monoterpenoids being the rest, wherein the bornyl acetate content is at least 10.0% relative to the total terpenes taken in certain proportions, and a pharmaceutically acceptable carrier and/or excipient, **characterized in that** the composition is orally administered to mammals, including a human, in an effective amount.

2. The pharmaceutical according to claim 1 for treating tumor diseases of various geneses, said pharmaceutical being represented by the pharmaceutical composition Abisilin which may be in the form of a solution, tablets, gelatin capsules, pills, syrups, suspensions, powders, emulsions, granulates, microspheres, or nanospheres or in other dosage forms useful for a controlled release of the active ingredients of the pharmaceutical.

3. The pharmaceutical according to claim 1 or 2 wherein, for inducing angiogenesis system inhibition and for causing the direct antitumor, antirecurrent, and antimetastatic effects and the associated activation of the endogenous apoptosis system, the pharmaceutical composition is administered orally to mammals including a human suffering from tumor diseasesevery day in a dose of 80 to 100 mg per kilogram of body weight.

4. The pharmaceutical according to claim 1, 2, or 3 wherein the pharmaceutical composition Abisilin is used together with other pharmaceutically active substances (antibiotics, cytostatics, and others).
